(19) Europäisches Patentamt
European Patent Office
Office européen des brevets

(11) **EP 4 160 614 A1**

(12) **EUROPEAN PATENT APPLICATION**

(43) Date of publication:
**05.04.2023 Bulletin 2023/14**

(21) Application number: **21206949.6**

(22) Date of filing: **08.11.2021**

(51) International Patent Classification (IPC):
**G16H 50/20** (2018.01)   **G16H 50/30** (2018.01)
**G16H 50/70** (2018.01)

(52) Cooperative Patent Classification (CPC):
**G16H 50/70; G16H 50/20; G16H 50/30**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.10.2021 US 202117493818**

(71) Applicant: **Koa Health B.V.**
**08018 Barcelona (ES)**

(72) Inventors:
 • **Buda, Teodora Sandra**
  **08019 Barcelona (ES)**

 • **Garriga Calleja, Roger**
  **08470 Barcelona (ES)**
 • **Guerreiro, João**
  **1600-630 Lisbon (PT)**
 • **Omaña Iglesias, Jesus Alberto**
  **08019 Barcelona (ES)**
 • **Matic, Aleksandar**
  **17310 Lloret de Mar (ES)**

(74) Representative: **Herrmann, Uwe**
**Lorenz Seidler Gossel**
**Rechtsanwälte Patentanwälte**
**Partnerschaft mbB**
**Widenmayerstraße 23**
**80538 München (DE)**

(54) **SYSTEM AND METHOD FOR TRIGGERING MENTAL HEALTHCARE SERVICES BASED ON PREDICTION OF CRITICAL EVENTS**

(57)    A system for triggering mental healthcare services based on prediction of critical events receives both structured data and unstructured data about mental healthcare patients. Feature extraction is performed, thereby generating records of structured data, and strings of vectors of unstructured data. The system makes a quality assessment about the structured data, and a quality assessment about the unstructured data. In a model selection step, the system selects one model out of a plurality of selectable models, where the selection is made based on the quality assessments made. The selected model is trained with records of structured data and with strings of vectors of unstructured data. New real-time data is supplied to the trained model so that the trained model predicts whether a crisis event is likely to occur. If the system predicts that a crisis event is likely to occur, then the system outputs an alert.

SYSTEM FOR TRIGGERING
MENTAL HEALTHCARE SERVICES
FIG. 13

**Description**

TECHNICAL FIELD

The described embodiments relate generally to a system that predicts that a mental health critical event will occur in a mental healthcare environment, and to related structures and methods.

BACKGROUND INFORMATION

Mental problems represent the third most common reason to visit a health center, often leading to diagnosed disorders. Typically, the demand for mental health services is triggered by a mental health crisis. In the mental health crises, the patient is no longer able to function effectively in the community, or there is a risk that the patient might hurt himself/herself or others. Such mental health crises situation are commonly suffered by patients diagnosed with psychotic, personality or severe mood disorders, however they also occur in patients diagnosed with less severe disorders or even non-diagnosed individuals under stressful situations. Acute mental crises include severe self-harm, delusions or suicide attempts and often require hospitalization. When such a serious crises event occurs, a large quantity of resources typically needs to be made available to treat the patient, and this results in a high cost for the mental facility.

Compared with outpatient care, inpatient care is typically more costly for the healthcare system and can have a negative impact on the patient's cognitive and psychosocial state as well as on the quality of their life. Clinical treatment can be very efficacious in mitigating symptoms and risks of relapse. However, success in preventing relapse and hospitalization depends greatly on when the deterioration patient clinical condition is detected, i.e., earlier detection and earlier treatment is typically associated with better outcomes measured in terms of cost, outcome and patient experience. Patients may not present to mental health services with the early warning signs of relapse, thus specialist services must often manage more complex cases with higher clinical acuity and accumulated psychosocial issues that add further complication.

To identify which patients are at an unacceptably high risk of having a mental health crises and consequently require urgent treatment currently typically requires medical personnel to review patient files and notes in order to make an assessment. There often is a huge number of patients per nurse and clinician. Additionally, there are a great many variables that can be used to make the assessment. Humans are not so good taking into account several variables at the same time. Moreover, with the increasing use of portable electronic devices and with the advent of electronic health records, there is now an increasing amount of patient data being generated and collected by physicians and hospitals. In the mental healthcare environment, there is a need to use and analyze the large amount of clinical data collected, but to do so in an efficient and reliable manner.

SUMMARY

A system for triggering mental healthcare services based on prediction of mental health critical events includes a monitoring unit, a quality assessment unit, a model selection unit, a feature extraction unit, a crisis prediction unit, and an output unit. The system receives and analyzes information about mental healthcare patients. That information is received in both structured data form as well as in unstructured data form. The system carries out a novel method. In a data ingestion and preprocessing step of the method, the system receives an amount of structured data. The structured data includes information about each patient of a plurality of patients. That information may include information such as demographics information, information regarding office visits made by the patient, information on diagnoses made regarding the patient, and information about any events in which the patient was hospitalized. Feature extraction is performed on the received structured data, thereby generating a plurality of records of structured data. Also in the data ingestion and preprocessing step, an amount of unstructured data is received into the system. An example of an amount of unstructured data is a textual note, such as a handwritten note or a typed note, where the content of the note relates to a patient. The unstructured data includes information about at least some patients of the plurality of patients. Feature extraction is performed on the received unstructured data, thereby obtaining a plurality of strings of vector values.

Next, in an assessment of data quality step, the system makes a quality assessment about the structured data received, and makes a quality assessment about the unstructured data received. Next, in a filtering and model selection step, the system selects one model out of a plurality of selectable models. In a typical example, each model of the plurality of selectable models is stored on the system. The selection is made based at least in part on the quality assessment made on the structured data and on the quality assessment made on the unstructured data. In one example, a first of the selectable models is a relatively simple rule-based decision tree model that is not a neural network model, and a second of the selectable models is a relatively complex machine learning model such as a neural network model. Next, in a model training step, if the selected model requires training, it is trained using at least some of records of structured data obtained in the feature extraction operation described above, and using a least some of the strings of vector values obtained in the feature extraction operation described above. In this way, the selected model is usable to predict whether a predetermined crisis event will likely occur. The system as trained is then used, and over time it ingests further structured and unstructured data about patients. Next, in a crises detection step, both structured data (for example, newly incoming real-time structured data) as well as unstructured data (for example, newly incoming real-time unstructured data) are supplied to the selected model so that the model constantly predicts whether a crisis event is likely to occur. If the system predicts that a crisis event is likely to occur, then the system outputs an alert, where the alert is indicative of the prediction of the crisis event by the system. This alert may, for example, be an email sent by the system to the treating physician where the email indicates that a particular patient is likely to suffer a crisis event in the next two weeks.

In another example, in the data ingestion step, an amount of structured data is received into the system, and an amount of unstructured data is received onto the system. At this stage, feature extraction is not performed on the structured data or on the unstructured data, but rather in the assessment of data quality step the system makes a quality assessment about the structured data and makes a quality assessment about the unstructured data. Depending on the result of the quality assessment of the structured data, feature extraction is performed or is not performed on the structured data and the resulting records of structured data are used in a subsequent model training step. If, for example, the result of the quality assessment of the structured data indicates that the structured data is of low quality, then feature extraction need not be performed and is not performed on the structured data, and the results of such feature extraction are not used in subsequent model training. Likewise, depending on the result of the quality assessment of the unstructured data, feature extraction is performed or is not performed on the unstructured data and the resulting strings of 60-dimensional vector values are used in a subsequent model training step. If, for example, the result of the quality assessment of the unstructured data indicates that the unstructured data is of low quality, then feature extraction need not be performed and is not performed on the unstructured data, and the results of such feature extraction is not used in subsequent model training. In the model selection step, the system selects one model out of a plurality of selectable models. The selection is made based at least in part on the quality assessment made on the structured data and on the quality assessment made on the unstructured data. The type of model selected may depend on whether the results of feature extraction on structured data are available for model training, and on whether the results of feature extraction on unstructured data are available for model training. The simplest and yet adequately effective model for the type of data available is chosen. Next, in the model training step, if the selected model requires training then the selected model is trained using the results of the feature extraction on structured data, if any, and using the results of feature extraction on unstructured data, if any. In this way, if the selected model is a model that requires training, the selected model is trained to predict whether a predetermined crisis event will likely occur. The system is then used, and over time it ingests further structured and unstructured data about patients. In a crisis detection step, both structured data (for example, newly incoming real-time structured data) as well as unstructured data (for example, newly incoming real-time unstructured data) are supplied to the selected model so that the model constantly outputs a prediction of whether a mental health crisis event is likely to occur. If the system predicts that a mental health crisis event is likely to occur, then the system outputs an alert, where the alert is indicative of the prediction of the mental health crisis event by the system.

Further embodiments and structures and methods and techniques are described in the detailed description below. This summary does not purport to define the invention. The invention is defined by the claims.

BRIEF DESCRIPTION OF THE DRAWINGS

The accompanying drawings, where like numerals indicate like components, illustrate embodiments of the invention.

FIG. 1 is a simplified diagram of a first example of a system for triggering mental healthcare services based on prediction of critical events.
FIG. 2 is a diagram of a table of demographics information about mental healthcare patients.
FIG. 3 is a diagram of a table that records office visits.
FIG. 4 is a diagram of a table of diagnosis information.
FIG. 5 is a diagram of a table of hospitalization information.
FIG. 6 is a diagram that illustrates the unstructured data in the system diagram of FIG. 1.
FIG. 7 is a diagram of a data compilation table that compiles structured data.
FIG. 8 is a diagram that illustrates one example of how a textual note is converted into a string of 60-dimensional vector values.
FIG. 9 is a table that illustrates how a PCM value is calculated for each patient, and then how a data quality assessment value is determined for each patient, and then how a single quality assessment value for the structured data is determined.
FIG. 10 is a table that illustrates how a PNM value is calculated for each patient, and then how a data quality assessment value is determined for each patient, and then how a single quality assessment value for the unstructured data is determined.

FIG. 11 is a table that illustrates how the selection of the model is made in the model selection step illustrated in the system diagram of FIG. 1.

FIG. 12 is a simplified diagram of a second example of a system for triggering mental healthcare services based on prediction of critical events.

FIG. 13 is a system-level block diagram of a system for triggering mental healthcare services based on prediction of critical events.

## DETAILED DESCRIPTION

Reference will now be made in detail to some embodiments of the invention, examples of which are illustrated in the accompanying drawings.

In a mental healthcare clinical setting, data and information about a mental healthcare patient and the patient's condition and treatment is generated and recorded in different forms. A first form of such data is referred to here as "structured data." For each informational element in a list, on a form, or in a table, there is a corresponding informational value to be recorded. If this informational value is known or gathered or determined in the clinical setting, then it is recorded in the proper place in association with an indication of its corresponding informational element. An informational element about a patient may, for example, be the date upon which a specified patient interfaced with a healthcare professional in a clinical setting as a formal office visit. The informational value may be recorded either by the patient, or by a doctor, or by another individual. There may, for example, be an electronic form served to an electronic device being used by a licensed medical professional, where the form includes a field that is labeled office visit. The licensed medical professional may use the electronic device such as a cellular telephone or a laptop computer or a desktop computer to access the form, and to type into the field the date of the office visit. The date of the office visit is the informational value. For each such informational value that may be entered, there is an associated descriptive informational element. The informational value is recorded in association with the informational element. Such data is referred to as structured data.

A second form of data pertaining to a mental healthcare patient and the patient's condition and treatment is referred to here as "unstructured data." An example of unstructured data is a textual note, such as a textual handwritten textual note written down by hand by a treating physician, or such as a textual note recorded by a patient. A note may be in a language such as English, in the form of sentences or sentence fragments. Such a textual note may be recorded in association with some referencing information, such as the date the note was recorded and the identity of the patient to whom the note pertains, but the bulk of the informational content of the note is in the form of prose language text without there being numerous sets of informational elements and associated informational values. An example of a textual note of unstructured data is "My dog passed away last week." An electronic form may be served, where the form includes a field into which a textual note can be typed. The author of the textual note or another person can enter the textual note into the field. A textual handwritten note, for example, may be read by a person other than the author and then typed into the field of the form.

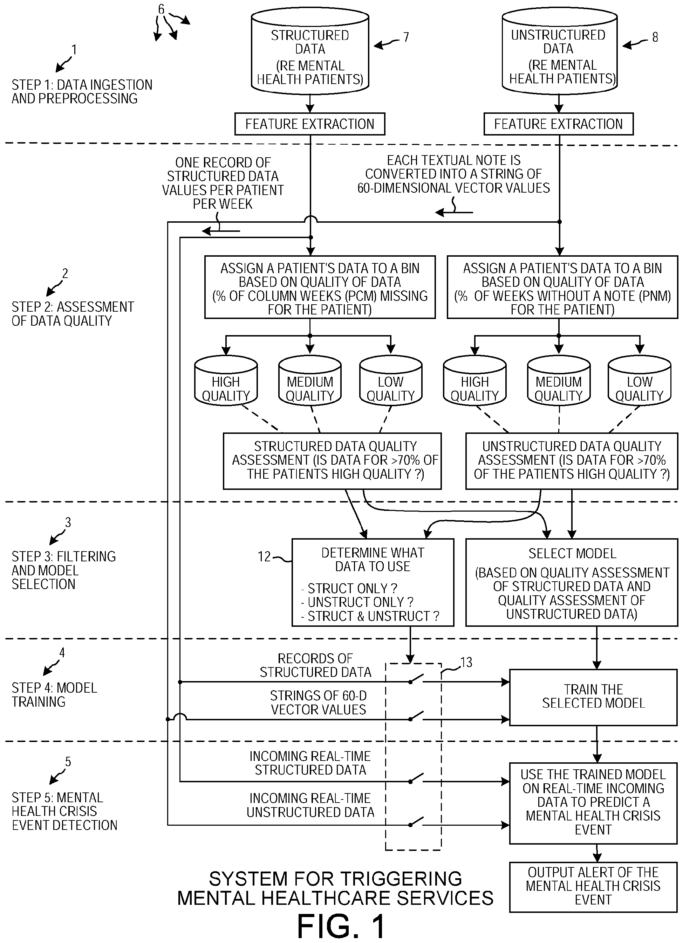

FIG. 1 is a simplified diagram of a first example of a system 6 for triggering mental healthcare services based on prediction of critical events. The diagram outlines and illustrates several steps of a novel method including: 1) a first step 1 titled "data ingestion and preprocessing," 2) a second step 2 titled "assessment of data quality," 3) a third step 3 titled "filtering (optional) and model selection," 4) a fourth step 4 titled "model training," and 5) a fifth step 5 titled "crisis detection."

In the first step 1, data is collected, generated, or retrieved and is then ingested into the system 6 so that it is stored electronically in the system 6. The words "ingested into" means "received into". The data received into the system includes both structured data 7 as well as unstructured data 8. In one example, the system 6 includes a web server that serves web pages to electronic devices of users of the system 6, so that the users can in turn use browsers on their electronic devices to enter data, both structured and unstructured, into the system 6 by filing in blanks, fields and queries in the web pages.

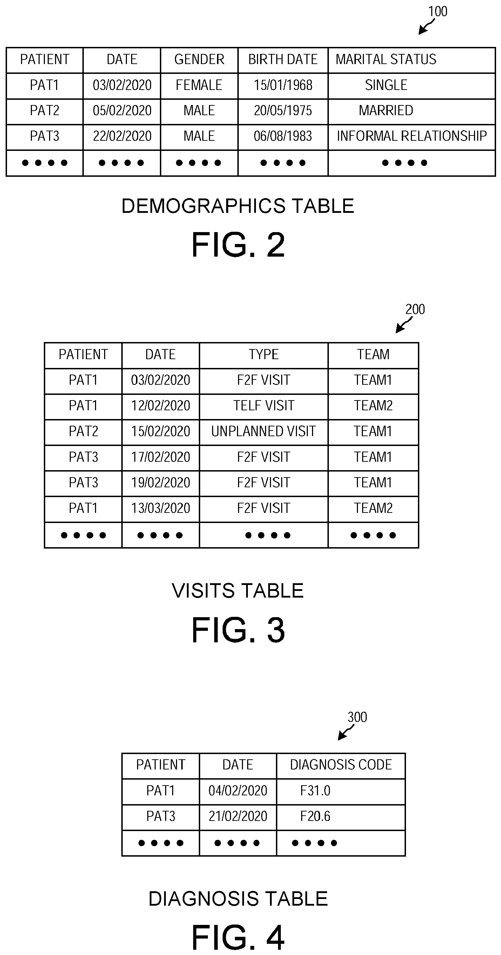

FIG. 2 is a diagram of a table 100 of demographics information about patients. It is a table of structured data as that data is stored on the system 6. The table 100 includes columns and rows. At the top of each column is listed an informational element. The informational elements of the columns are "PATIENT", "DATE", "GENDER", "BIRTH DATE", and "MARITAL STATUS". For an individual patient, identified as "PAT1", the date (date of entry of the diagnostics information) is "03/02/2020", the gender of the patient is "FEMALE", the birth date of the patient is "15/01/1968", and the marital status of the patient is "SINGLE". For each patient of a set of patients, this demographic information is received into and stored in the system in the diagnostics information table 100.

FIG. 3 is a is a diagram of a table 200 that records office visits. It is a table of structured data as that data is stored on the system 6. The table 200 includes columns and rows. At the top of each column is listed an informational element. The informational elements of the columns are "PATIENT", "DATE", "TYPE", and "TEAM". Each row records information about an individual office visit. The first row, for example, records information about an office visit of the patient "PAT1". The date of the visit is recorded as "03/02/2020." The recorded type of visit was "F2F VISIT", and the team of healthcare professionals that handled the visit is indicated to be "TEAM1". The system collects and recorded information about many such office visits, and for each visit there is a corresponding row of information in table 200.

FIG. 4 is a diagram of a table 300 of diagnosis information. It is a table of structured data as that data is stored on the system 6. The table 300 includes columns and rows. At the top of each column is listed an informational element. The informational elements of the columns are "PATIENT", "DATE", and "DIAGNOSIS CODE". Each row records information about an individual diagnosis that was made. The first row, for example, records information about a diagnosis related to the patient "PAT1", that was made on the date "04/02/2020". The entry "F31.0" indicates the type of diagnosis that was made. For each patient of a set of diagnosis, this diagnosis table 300 includes an entry that is stored as a row in the table.

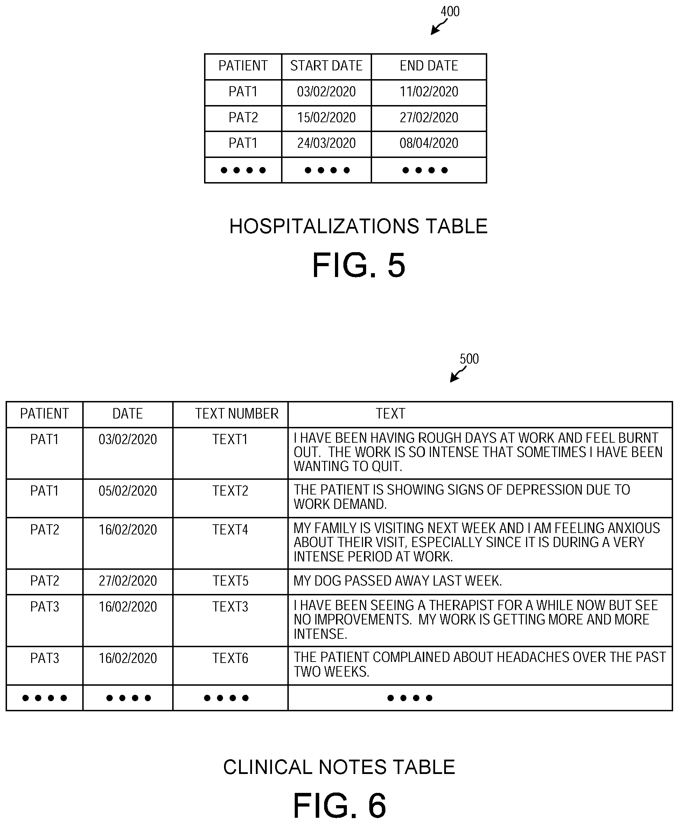

FIG. 5 is a diagram of a table 400 of hospitalization information. It is a table of structured data as that data is stored on the system 6. The table 400 includes columns and rows. At the top of each column is listed an informational element. The informational elements of the columns are "PATIENT", "START DATE", and "END DATE". Each row records information about a hospital stay of a patient. The first row, for example, records information about a hospital stay of the patient "PAT1", that started on "03/02/2020", and that ended on "11/02/2020". For each hospital stay of a patient whose clinical treatment is being tracked by the system 6, there is a row of information about the hospital stay recorded in the table 400.

FIG. 6 is a diagram that illustrates the unstructured data illustrated in the system diagram of FIG. 1. Although the unstructured data is recorded and stored in the system in the form of a table 500 as shown, each actual textual note stored is an amount of unstructured data. As can be seen, the table 500 stores six different textual notes. For each note, the table stores information about the associated patient, the date the textual note was made or recorded, and an identifying text number. The row of the table 500, for example, stores a short textual message for the patient "PAT2", made on "27/02/2020", where the textual message is assigned an identifier number of "TEXT5". The textual note is "MY DOG PASSED AWAY LAST WEEK."

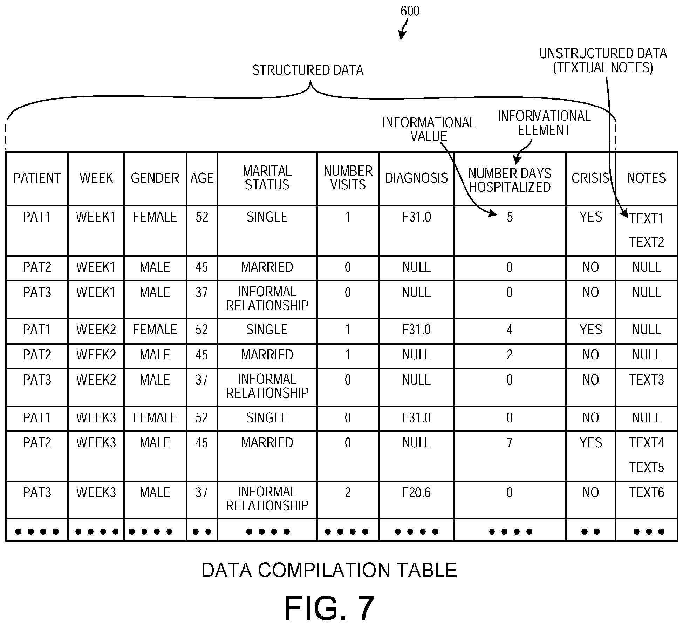

In the data ingestion and preprocessing step 1 of FIG. 1, the data of the tables of FIG. 1 through FIG. 6 is compiled into a data compilation table 600 as shown in FIG. 7. The table 600 records and references both structured data as well as unstructured data. Each row of the table 600 is referred as a "record" of structured data. For each patient being tracked by the system, for each week of a set of weeks, the table 600 includes one record. The indicated week of "WEEK1" is, in this example, the calendar week from and including February 2, 2020 until and including February 8, 2020. For that week designated as WEEK1, the table 600 includes a record for each of three patients "PAT1", PAT2" and "PAT3". The first row of the table records the record for patient "PAT1" for "WEEK1". The record includes, for each of a set of informational elements, the corresponding informational values. A recording of "NULL" indicates that no informational value for the associated informational element was recorded and stored. The first record in the table 600 indicates that for patient "PAT1", during the week of "WEEK1", the following structured data is recorded: GENDER: FEMALE, AGE: 52, MARITAL STATUS: SINGLE, NUMBER OF VISITS: 1, DIAGNOSIS: F31.0, NUMBER DAYS HOSPITALIZED: 5, CRISES: YES, and there are two associated notes identified by their identifiers "TEXT1" and TEXT2". The value of the informational element "CRISIS" may be entered by the healthcare professional or hospital staff and/or it may be generated by the system 6 in accordance with a rule. For example, the system 6 may define a crisis as occurring if either one or more of the following occurred during the indicated week: a hospital stay of more than a predetermined number of days, or a diagnosis of one of a predetermined serious conditions, or the patient went in for unscheduled office visit during the week. By the compilation and creation of the table 600 of FIG. 7 is referred to as "feature extraction". In this way, numerous records of structured data are created. These records are created in real time as the system 6 operates. During each successive week, more records are generated.

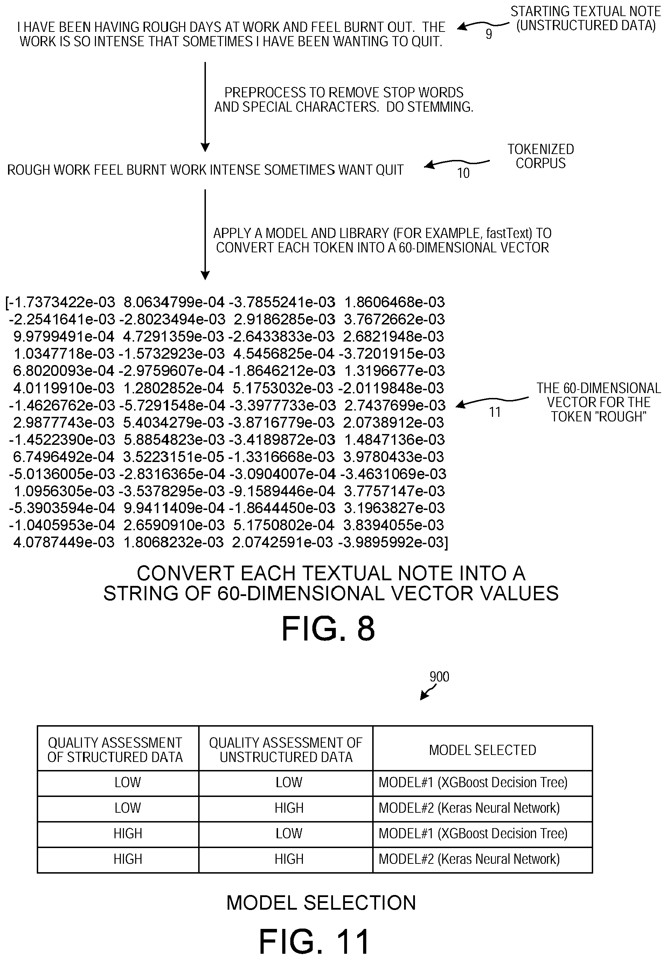

The data ingestion and preprocessing step 1 of FIG. 1 further includes "feature extraction" performed on the unstructured data. FIG. 8 is a simplified diagram of one example of such feature extraction. At the top of FIG. 8, the textual note 9 (TEXT1) of the table 500 of FIG. 6 is set forth. This is one of the two textual notes generated in WEEK1 for patent PAT1 as indicated by the table 600 of FIG. 7. The system 6 preprocesses the text of note 9 (TEXT1) to remove certain stop words and special characters. For example, the punctuation characters are removed. For example, the word "AT" is considered to have little value and is therefore removed as being a form of a stop word. In addition, stemming is performed to replace certain words with their stem words. For example, the word "wanting" in the original text of TEXT1 is replaced with its stem word "want". The result is what is sometimes called a "tokenized corpus". The tokenized corpus 10 in the example of FIG. 8 is "ROUGH WORK FEEL BURNT INTENSE SOMETIMES WANT QUIT."

Next, a model and library is applied to convert the tokenized corpus 10 into a corresponding string of 60-dimensional vectors values. In the present example, the open-source model and library called "fastText" may be employed. The program was developed by Facebook's AI Research lab, and is open-source available with a BSD license. The program is downloaded, installed, and run on the system 6 in order to convert each token of the tokenized corpus 10 into a corresponding 60-dimensional vector value. For example, as illustrated in FIG. 8, the first token "ROUGH" in the tokenized corpus 10 is converted into the illustrated set of numbers 11 that is referred to as a 60-dimensional vector value.

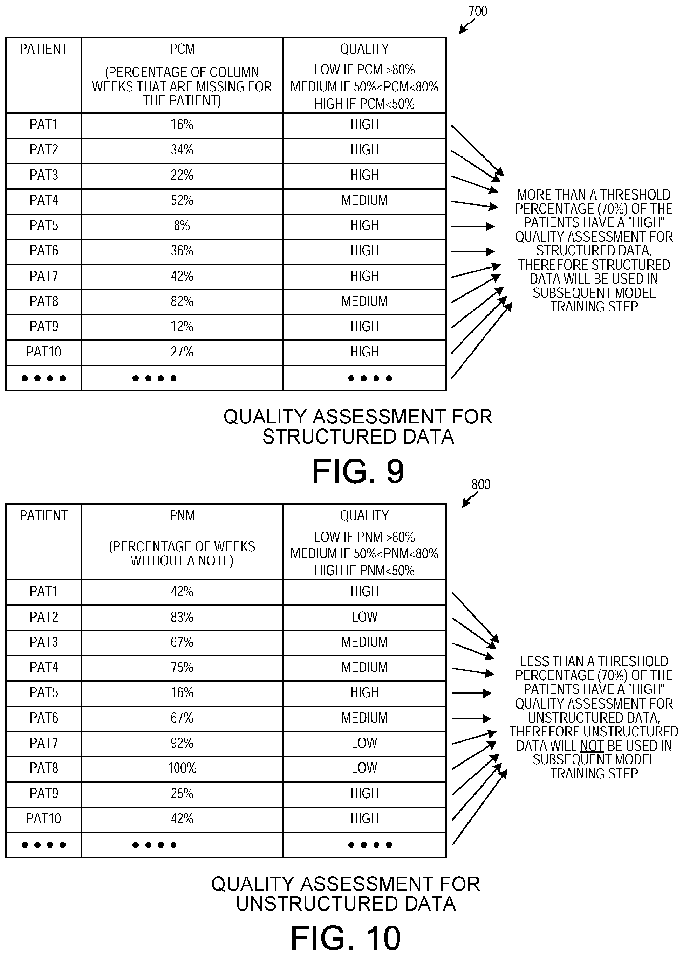

In the assessment of data quality step 2 of FIG. 1, a quality assessment is made about the structured data stored in the system, and a quality assessment is made about the unstructured data stored in the system. In one example, the quality assessment about the structured data is performed as follows. The structured data in the data compilation table of FIG. 6 for each patient is considered, but is considered separately. For each patient, all the records (each record is for one week) are considered, and the total percentage of "column week" values missing in the table for that patient is determined. The term "column week value" here refers to an informational value in one of the informational elements of a row in the table for that patient being considered. The table of FIG. 7 is but an illustrative example. A typical data compilation table would have many more columns than the table illustrated in FIG. 7, and many of the informational value boxes (column week values) would be unoccupied (NULL). For each patient, the percentage of column week values (PCM) that are missing from the patient's structured data weekly records is determined. FIG. 9 is a table that illustrates a set of calculated PCM numbers for patients PAT1 through PATIO. Next, each PCM value is characterized as being of either high quality, or medium quality, or low quality. In the example illustrated in FIG. 9, if the PCM value is greater that eighty percent, then so many of the informational values are missing that the patient's structured data is deemed to be of low quality. A value of "LOW" is entered into the row of the table corresponding to the patient. If, however, the PCM value is between fifty percent and eighty percent, then the patient's structured data is deemed to be of medium quality. A value of "MEDIUM" is entered into the row of the table corresponding to the patient. If, however, the PCM value is less than fifty percent, then not much of the patient's structure data is missing and the patient's structured data is deemed to be of high quality. Such an assessment of quality, based on the PCM value for each patient, is performed for each patient. In the illustrated example, these values are the values in the rightmost column of the table 700 of FIG. 9. In a variation, the two percentages (in the above example, the fifty percent value and the eighty percent value) used to determine whether a patient's structured data is of high quality, medium quality or low quality are changed, selected and tuned depending on system/hospital requirements.

Next, a rule is applied to determine whether the structured data of all the patients as a whole will be considered to be of low quality, or of high quality. In the illustrated example of FIG. 9, if more than a threshold percentage (in this case, seventy percent) of the quality indications in the right column of the table of FIG. 9 are "HIGH", then the quality assessment for the structured data as a whole is deemed to "HIGH", otherwise the quality assessment for the structured data as a whole is deemed to "LOW".

In one example, the quality assessment about the unstructured data is performed as follows. For the unstructured data of each patient, a "percentage of weeks without a note" value (a PNM value) is generated. Each patient is being tracked by the system 6 during a time period involving one or more weeks. A particular patient may, for example, be tracked by the system 6 over a period of fifty-five consecutive weeks. The PNM value is generated by consulting the data compilation table of FIG. 7. The number of the rows in the table for the patient that have no textual note entry is divided by the total number of rows in the table for the patient, and the result is the PNM value for that patient. This PNM value determination is performed for each patient, and the results are entered into the second column of the table 800 of FIG. 10. Next, each PNM value is characterized as being of either high quality, or medium quality, or low quality. In the example illustrated in FIG. 10, if the PNM value is greater that eighty percent, then there are so few notes for that patient that the patient's unstructured data is deemed to be of low quality. A value of "LOW" is entered into the row of the table corresponding to the patient. If, however, the PNM value is between fifty percent and eighty percent, then the patient's unstructured data is deemed to be of medium quality. A value of "MEDIUM" is entered into the row of the table corresponding to the patient. If, however, the PNM value is less than fifty percent, then there are many notes of the patient and the patient's unstructured data is deemed to be of high quality. Such an assessment of quality, based on the PNM value for each patient, is performed for each patient. In the illustrated example, these values are the values in the rightmost column of the table 800 of FIG. 10. Next, a rule is applied to determine whether the unstructured data of all the patients as a whole will be considered to be of low quality, or of high quality. In the illustrated example of FIG. 10, if more than a threshold percentage (in this case, seventy percent) of the quality indications in the right column of the table of FIG. 9 are "HIGH", then the quality assessment for the unstructured data as a whole is deemed to "HIGH", otherwise the quality assessment for the unstructured data as a whole is deemed to "LOW".

Next, in the filtering (optional) and model selection step 4 of FIG. 1, a model is selected based at least in part on the results of the prior quality of data assessment step 2. The model to be selected is a model that determines, based on input data, whether a particular crisis event is predicted to occur. The novel system 6 of FIG. 1 chooses and selects the model that is, according to a metric, the best model (of a set of selectable models) to use in the particular circumstance involving the particular determined quality of structured data, and the particular determined quality of unstructured data. There is a first model and a second model, where the first model is a relatively simple model as compared to the second model.

In the presently described example, the first model is an open-source machine learning program and library called XGBoost, that is available from the developer The XGBoost Contributors with an Apache License 2.0. This model and program uses a gradient boosting decision tree, rather than a more complex neural network.

In the presently described example, the second model is an open-source neural network program and library called Keras, that is available from MIT with an MIT license. This model and program uses a neural network that must be trained, rather than the relatively simple decision tree of XGBoost.

FIG. 11 is a table 900 that illustrates how the selection of the model is made in step 3. As indicated by the table 900, if the determined quality of the unstructured data is high, then the second model (the Keras neural network model) is selected, otherwise the first model (the XGBoost model) is selected.

Next, in the model straining step 4 of FIG. 1, the selected model is trained. In the present example, the selected model is the second model (the neural network model). The result value that the neural network model is being trained to predict is whether a crisis event will occur in the next two week period. Each of the records of structured data supplied to the neural network in this training step includes a crisis event informational value. The crises event value is a binary value that can have a value of "YES", a value of "NO". In this case, the system 6 makes sure that there is a value in each row of the table 600 of FIG. 7 for the crises event column. If a value of "YES" is not given, or cannot be determined, then the value of "NO" is assigned.

All the collected records of structured data can be supplied to the model for training, or only some of the collected records can be supplied. Likewise, all of the collected strings of 60-dimensional vector values can be supplied to the model for training, or only some of the collected strings of 60-dimensional vector values can be supplied. In the illustration of FIG. 1, blocks 12 and 13 represent this possible filtering of data that is supplied to the neural network model from training. For example, if the assessed quality of the unstructured data is high where the assessed quality of the structured data is low, then the records of structured data may not be supplied to the model for training.

After the selected model has been trained in step 4, the trained model is then used in the crises detection step 5 of FIG. 1. As time goes by and as additional information for patients is ingested into the system 6, the additional real-time records of structured data and strings of 60-dimensional vector values are supplied to the trained model. From this new input data, the trained model constantly determines whether a crisis event is predicted to occur in the next two weeks. The results value output by the trained neural network is a binary value that indicates "Yes" or "No" as to whether a crises event is predicted to occur in the next two weeks. If such a crises event is predicted to occur, then the neural network also outputs an indication of the associated patient. If the system 6 determines that a crisis event is likely to occur in the next two weeks, then an alert is output by the system 6. In one example, the alert is an email communication to the treating physician and to that physician's office staff.

Avoiding Unnecessary Feature Extraction: Although the example of the method carried out by the system 6 as described above in connection with FIGS. 1-11 involves performing feature extraction on both structured data as well as unstructured data, in another example unnecessary feature extraction is avoided. In step 1, structured data and unstructured data is received into the system, but no feature extraction is performed. In the assessment of data quality step, the system makes a quality assessment about the structured data and makes a quality assessment about the unstructured data. Depending on the result of the quality assessment of the structured data, feature extraction is performed or is not performed on the structured data and the resulting records of structured data are used in a subsequent model training step. If, for example, the result of the quality assessment of the structured data indicates that the structured data is of low quality, then feature extraction need not be performed and is not performed on the structured data, and the results of such feature extraction are not used in subsequent model training. Likewise, depending on the result of the quality assessment of the unstructured data, feature extraction is performed or is not performed on the unstructured data and the resulting strings of 60-dimensional vector values are used in a subsequent model training step. If, for example, the result of the quality assessment of the unstructured data indicates that the unstructured data is of low quality, then feature extraction need not be performed and is not performed on the unstructured data, and the results of such feature extraction is not used in subsequent model training. The system selects one model out of a plurality of selectable models. Model selection is based at least in part on the quality assessment made on the structured data and on the quality assessment made on the unstructured data. The type of model selected depends on whether the results of feature extraction on structured data are available for model training, and on whether the results of feature extraction on unstructured data are available for model training. The simplest and yet effective model for the type of training data available is chosen. If the added complexity and computational demands on the system associated with use of a relatively complex model would not lead to appreciably better crisis prediction results for the particular data available (structured data available, and unstructured data available), then a relatively less complex model is selected so as to speed system operation and to reduce computational demands on the system. Next, in the model training step, the selected model is trained using the results of the feature extraction on structured data, if any, and on the results of feature extraction on unstructured data, if any. The trained system is then used, and over time it ingests further structured and unstructured data about patients. In a crisis detection step, both structured data (for example, newly incoming real-time structured data) as well as unstructured data (for example, newly incoming real-time unstructured data) are supplied to the trained model so that the trained model constantly predicts whether a crisis event is likely to occur. If the system predicts that a crisis event is likely to occur, then the system outputs an alert, where the alert is indicative of the prediction of the crisis event by the system.

Another Way To Assess Data Quality In Step 2: The embodiment of the system 6 described above is but one illustrative example that involves one particular example of the "assessment of data quality" step 2. The quality of the structured data and the quality of the unstructured data can be assessed in other ways. In another example, the quality of the unstructured data is assessed using a topic modeling based quality assessment as described below. The unstructured data is classified into three quality bins as follows. For each patient and each file corresponding to a week's notes of data available, LDA (Latent Dirichlet Allocation) is applied and topic coherence is computed for each file, where a file is aggregation of each patient's notes for that week as shown below in Equation 1.

$$File(week, patient) = U \ Notes(week, patient) \quad (Eq. \ 1)$$

Next, average topic coherence is computed across all notes per patient as shown below in Equation 2.

$$AvgTC(patient) = \frac{\sum_{Week=0}^{NumberOfWeeks(patient)} TC \ (File(week,patient))}{NumberOfWeeks(patient)} \quad (Eq. \ 2)$$

This average topic coherence score gives an indication of how good the quality of the notes are per patient. The underlying assumption is that for some patients and for some doctors, the notes can be of better quality (for example, for more severely-affected patients). In the case that there are not sufficient notes per patient and a weekly aggregation of notes would give an empty document, the TC (File(week,patient)) is set to zero. Next, the topic coherence average scores are normalized between zero and one across the available scores across all patients. The unstructured data per patient is then classified into the following bins: (A) Low Quality: all notes of patients with an average topic coherence of <0.25 (Q1); (B) Medium Quality: all notes of patients with an average topic coherence between 0.25 and 0.5 (Q2); and (C) High Quality: all notes of patients with an average topic coherence above 0.5 (Q3 and Q4). In a variation, these percentages are tuned depending on system/hospital requirements

In one variation, only the unstructured textual data is assessed and placed into the three quality bins, whereas the structured data is always used (for instance considering decision tree machine learning models that are less impacted by missing data points, structured data may be ingested at all times regardless of its quality).

In another variation, data is placed into bins based on the number of weeks of available clinician notes (for example, low quality for twelve weeks, medium quality for twenty-four weeks, or high quality for over forty weeks of notes available during a one year period of health records).

In yet another variation, a different evaluation metric than the topic coherence is used for evaluating the quality of the notes. For example, model perplexity is used as the evaluation metric.

Other Examples Of Model Selection Step 3: The embodiment of the system 6 described above is but one illustrative example that involves one particular example of step 3. In another example, the system filters out the data that does not correspond to system requirements. The system decides on whether to include the structured data and/or the unstructured data in model training step, and this decision is based on whether the structured data and the unstructured data available in the high quality bin is in majority (if over fifty percent of the available data is of high quality for each type of data, structured or unstructured).

In a variation, an optimal threshold is determined by manually training a ML model for each combination of input data based on tis quality (low, medium, high only, medium and high, low and high, etc.) and selecting the input data that provided the best results.

In yet another variation, based on given requirements from the hospital or other users of the system, only data of high quality may be used to train a ML model.

Other Ways To Detect Crises In Step 5: The embodiment of the system 6 described above is but one illustrative example that involves one particular example of using the trained model to predict a crisis event in step 5. In another example, the trained model is applied on incoming patient data, and the trained model outputs multiple prediction outputs. The prediction outputs are then placed in an ordered list. The entire ordered list can then be assessed by medical experts to determine whether patent contact is needed. In this case, the system 6 does not actually output an alert that is sent to the treating physician identifying one crisis event that is predicted to occur, but rather a medical expert such as the physician or hospital staff is supplied with the entire ordered list

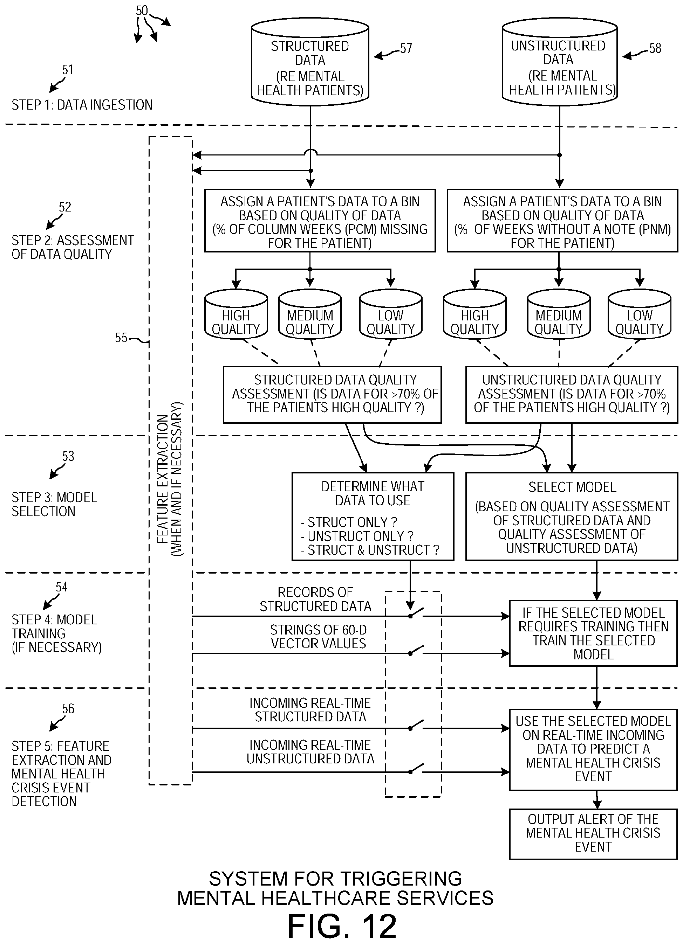

FIG. 12 is a diagram of a second example of a system for triggering mental healthcare services based on prediction of critical events. The system 50 carries out a method including the steps of data ingestion 51, data quality assessment 52, model selection 53, model training 54 as necessary, feature extraction 55 as necessary, and mental health crisis event detection 56. An amount of structured data 57 is received into the crisis event detection system. The structured data includes information about each patient of a plurality of patients. An amount of unstructured data 58 is also received into the system. The unstructured data includes information about each of at least some of the patients. The system 50 makes a quality assessment about the structured data received, and makes a quality assessment about the unstructured data received. In one example, the quality assessment of the structured data is made as described above in connection with FIG. 9 and the quality assessment of the unstructured data is made as described above in connection with FIG. 10. The system 50 stores a plurality of modes. Next, one model of the plurality of selectable models is selected, where the selection is based at least in part on the quality assessment made about the structured data and on the quality assessment made about the unstructured data. In one example, model selection is carried out using the relation illustrated in FIG. 11. Due to use of this relation in the selection of the model, the model selected is the least computationally complex model that at the same time for the data available results in adequate accuracy in the making of a mental health crisis event prediction. In the method being described in connection with FIG. 12, no feature extraction need have been done on any structured data or on any unstructured data up to this point in the method. Next, if the selected model is a type of model that requires training, then the model is trained. For example, feature extraction may be performed on some or all of the structured data 57 thereby generating records of structured data, and those records are then supplied the selected model to train it. At the same time, feature extraction may be performed on some or all of the unstructured data 58 thereby generating strings of sixtydimensional vector values, and those strings are supplied to the selected model to train it. If, however, the selected model is a model that does not require training, such as a more simple rule-based decision tree model, then no feature extraction and no training is performed. Next, in normal operation of the system in a mental health crisis event detection step of the method, new incoming real-time data (both structured data as well as unstructured data) is received into the system over time. As system operates and the new incoming data is received over time, feature extraction is performed on the new incoming data as required by the selected model, and also may be performed on historical stored data as required by the selected model. The results of the feature extraction is supplied to the selected model so that the model constantly over time is updated with data and works to predict mental health crisis events. In the illustration of FIG. 12, the feature extraction block 55 is shown in dashed lines spanning the data assessment, model selection, model training, and crisis event detection steps in order to indicate that feature extraction may be performed at any of these points in the method. In one example, the smallest amount feature extraction possible is performed because only that feature extraction required by the selected model is performed.

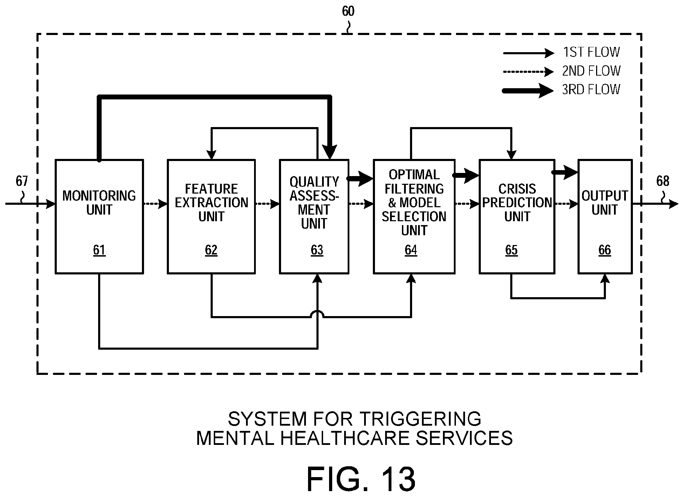

FIG. 13 is a system-level block diagram of a system 60 for triggering mental healthcare services based on prediction of critical events. The system 60 includes a monitoring unit 61, a feature extraction 62, a data quality assessment unit 63, an optimal filtering and model selection unit 64, a crisis prediction unit 65, and an output unit 66. As indicated by the legend on the diagram, the thin solid-line arrows indicate a first process flow through the units, whereas the dashed-line arrows indicate a second process flow through the units, whereas the thicker solid-line arrows indicate a third process flow through the units. The incoming arrow 67 represents the receiving of incoming structured and unstructured data. In one example, this data is received onto the system 60 from a database, such as a database maintained at a hospital. Raw data is loaded into the hospital database by other means. The arrow 68 represents the outputting of an alert, where the alert indicates that the system has predicted a mental health crisis event will likely occur in the next two weeks. In one example, the output is an email that is sent from the system 60 to the email address of a physician in order to alert the physician of the predicted mental health care crisis event. Each of the individual blocks 61-66 represents a discrete section or module or unit of computer programming code. This code is executed by a processor the system 60, and is stored in a computerreadable medium of the system, such as on a hard disk and/or in semiconductor memory. In some embodiments, use of the model selected requires no feature extraction at all including on the incoming real-time data used by the model in the prediction of the mental health crises event. In some embodiments, a feature extraction step is performed by a first legal entity such that the results of feature extraction are placed into a database maintained by the first legal entity, and then the results of that feature extraction are read out of the database and into a server maintained by a second legal entity such that the second legal entity uses and operates its server to carry out the remainder of the process steps of data quality assessment, model selection, and use of the model in predicting mental health crisis events.

Although certain specific embodiments are described above for instructional purposes, the teachings of this patent document have general applicability and are not limited to the specific embodiments described above. Accordingly, various modifications, adaptations, and combinations of various features of the described embodiments can be practiced without departing from the scope of the invention as set forth in the claims.

**Claims**

1. A method comprising:

(a) receiving an amount of structured data into a crisis event detection system, wherein the structured data includes information about each patient of a plurality of patients;
(b) receiving an amount of unstructured data into the crisis event detection system, wherein the unstructured data includes information about each of at least some patients of the plurality of patients;
(c) making a quality assessment about the data received in (a);
(d) making a quality assessment about the data received in (b);
(e) selecting one model of a plurality of selectable models, wherein the selection of (e) is based at least in part on the quality assessment made in step (c) and on the quality assessment made in step (d);
(f) performing feature extraction on at least some structured data or some unstructured data thereby obtaining results of the feature extraction; and
(g) supplying the results of the feature extraction obtained in (f) to the model selected in (e) so that the model makes a prediction of a mental health crisis event, and wherein (a) through (g) are performed by the crisis event detection system.

2. The method of Claim 1, wherein the at least some structured data or some unstructured data of (f) includes some of the amount of structured data received in (a).

3. The method of Claim 1, wherein the at least some structured data or some unstructured data of (f) includes some of the amount of unstructured data received in (b).

4. The method of Claim 1, wherein the at least some structured data or some unstructured data of (f) includes none of the structured data received in (a) and none of the unstructured data received in (b).

5. The method of Claim 1, wherein at least some of the feature extraction of (f) occurs prior to the selecting of the model in (e).

6. The method of Claim 1, wherein none of the feature extraction of (f) occurs prior to the selecting of the model in (e).

7. The method of Claim 1, wherein the plurality of selectable models includes a first model and a second model, wherein the first model is a machine learning model that requires training, and wherein the second model is a rule-based model that does not require training, wherein the model selected in (e) is the first model, the method further comprising: (h) using feature extraction results to train the model selected in (e).

8. The method of Claim 1, wherein the quality assessment made in (c) involves making, for each patient, a quality assessment of the structured data received in (a) for that patient, and then from the quality assessments of the patients determining a single overall quality assessment, wherein the single overall quality assessment is the quality assessment made in (c).

9. The method of Claim 8, wherein the quality assessment of the structured data received in (a) for a patient is determined based upon a percentage of the patient's structured data that is missing.

10. The method of Claim 1, wherein the quality assessment made in (d) involves making, for each patient, a quality assessment of the unstructured data received in (b) for that patient, and then from the quality assessments of the patients determining a single overall quality assessment, wherein the single overall quality assessment is the quality assessment made in (d).

11. The method of Claim 10, wherein the quality assessment of the unstructured data received in (b) for a patient is determined based on a measure of an amount of notes received into the system for the patient.

12. The method of Claim 10, wherein the quality assessment of the unstructured data received in (b) for a patient is determined using a Latent Dirichlet Allocation (LDA) model based topic coherence labels.

13. The method of Claim 1, wherein the crisis event detection system serves web pages usable to supply both the structured data received in (a) and the unstructured data received in (b) into to the crisis event detection system.

**14.** The method of Claim 1, further comprising:
(h) outputting from the crisis event detection system an alert, wherein the alert indicative of the prediction made in (g) of the mental health crisis event.

**15.** The method of Claim 1, further comprising:
(h) outputting from the crisis event detection system an electronic communication, wherein the electronic communication conveys an alert, wherein the alert is indicative of the prediction made in (g) of the mental health crisis event.

**16.** The method of Claim 1, wherein the feature extraction of (f) comprises generating a plurality of records, wherein each record includes a set of informational elements and a corresponding set of informational values, wherein one of the informational elements is a crisis event informational element, and wherein the informational value corresponding to the crisis event informational element indicates whether a mental health crises event occurred.

**17.** The method of Claim 1, wherein the feature extraction of (f) comprises generating a plurality of strings of multi-dimensional vector values.

**18.** A method, comprising:

(a) receiving an amount of structured data into a crisis event detection system, wherein the structured data includes information about each patient of a plurality of patients;
(b) performing feature extraction on the data received in (a) thereby obtaining a plurality of records of structured data;
(c) receiving an amount of unstructured data into the crisis event detection system, wherein the unstructured data includes information about each patient of the plurality of patients;
(d) performing feature extraction on the data received in (c) thereby obtaining a plurality of strings of vector values;
(e) making a quality assessment about the data received in (a);
(f) making a quality assessment about the data received in (c);
(g) selecting one model of a plurality of selectable models, wherein a first of the selectable models is a trained model, wherein a second of the models is a rule-based model that is not a trained model, wherein the selection of (g) is based at least in part on the quality assessment made in step (e) and on the quality assessment made in step (f); and
(h) supplying both structured data as well as unstructured data to the model selected in (g) so that the selected model outputs a prediction of a mental health crisis event.

**19.** A crisis event detection system comprising:

a monitoring unit for receiving an amount of structured data into the crisis event detection system, wherein the structured data includes information about each patient of a plurality of patients, wherein the monitoring unit is also for receiving an amount of unstructured data into the crisis event detection system, wherein the unstructured data includes information about each of at least some patients of the plurality of patients;
a quality assessment unit for making a quality assessment about the amount of structured data received by the monitoring unit and for making a quality assessment about the amount of unstructured data received by the monitoring unit;
a model selection unit for selecting one model of a plurality of selectable models, wherein the selection by the model selection unit is based at least in part on the quality assessment of the structured data made by the quality assessment unit and at least in part on the quality assessment of the unstructured data made by the quality assessment unit;
a feature extraction unit for performing feature extraction on at least some of structured data or unstructured data received by the monitoring unit thereby generating results of the feature extraction;
a crisis prediction unit for supplying the results of the feature extraction performed by the feature extraction unit to the one model selected by the model selection unit so that the one model makes a prediction of a mental health crisis event; and
an output unit for outputting from the crisis event detection system an alert that is indicative of the prediction of the mental health crisis event.

**20.** A method comprising:

(a) receiving an amount of structured data into a crisis event detection system, wherein the structured data includes information about each patient of a plurality of patients;

(b) receiving an amount of unstructured data into the crisis event detection system, wherein the unstructured data includes information about each of at least some patients of the plurality of patients;

(c) making a quality assessment about the data received in (a);

(d) making a quality assessment about the data received in (b);

(e) selecting one model of a plurality of selectable models, wherein the selection of (e) is based at least in part on the quality assessment made in step (c) and on the quality assessment made in step (d); and

(f) using the model selected in (e) to make a prediction of a mental health crisis event, and wherein (a) through (f) are performed by the crisis event detection system.

SYSTEM FOR TRIGGERING
MENTAL HEALTHCARE SERVICES

# FIG. 1

100

| PATIENT | DATE | GENDER | BIRTH DATE | MARITAL STATUS |
|---------|------|--------|------------|----------------|
| PAT1 | 03/02/2020 | FEMALE | 15/01/1968 | SINGLE |
| PAT2 | 05/02/2020 | MALE | 20/05/1975 | MARRIED |
| PAT3 | 22/02/2020 | MALE | 06/08/1983 | INFORMAL RELATIONSHIP |
| • • • • | • • • • | • • • • | • • • • | • • • • |

DEMOGRAPHICS TABLE

# FIG. 2

200

| PATIENT | DATE | TYPE | TEAM |
|---------|------|------|------|
| PAT1 | 03/02/2020 | F2F VISIT | TEAM1 |
| PAT1 | 12/02/2020 | TELF VISIT | TEAM2 |
| PAT2 | 15/02/2020 | UNPLANNED VISIT | TEAM1 |
| PAT3 | 17/02/2020 | F2F VISIT | TEAM1 |
| PAT3 | 19/02/2020 | F2F VISIT | TEAM1 |
| PAT1 | 13/03/2020 | F2F VISIT | TEAM2 |
| • • • • | • • • • | • • • • | • • • • |

VISITS TABLE

# FIG. 3

300

| PATIENT | DATE | DIAGNOSIS CODE |
|---------|------|----------------|
| PAT1 | 04/02/2020 | F31.0 |
| PAT3 | 21/02/2020 | F20.6 |
| • • • • | • • • • | • • • • |

DIAGNOSIS TABLE

# FIG. 4

400

| PATIENT | START DATE | END DATE |
|---------|-----------|----------|
| PAT1 | 03/02/2020 | 11/02/2020 |
| PAT2 | 15/02/2020 | 27/02/2020 |
| PAT1 | 24/03/2020 | 08/04/2020 |
| • • • • | • • • • | • • • • |

## HOSPITALIZATIONS TABLE

# FIG. 5

500

| PATIENT | DATE | TEXT NUMBER | TEXT |
|---------|------|-------------|------|
| PAT1 | 03/02/2020 | TEXT1 | I HAVE BEEN HAVING ROUGH DAYS AT WORK AND FEEL BURNT OUT. THE WORK IS SO INTENSE THAT SOMETIMES I HAVE BEEN WANTING TO QUIT. |
| PAT1 | 05/02/2020 | TEXT2 | THE PATIENT IS SHOWING SIGNS OF DEPRESSION DUE TO WORK DEMAND. |
| PAT2 | 16/02/2020 | TEXT4 | MY FAMILY IS VISITING NEXT WEEK AND I AM FEELING ANXIOUS ABOUT THEIR VISIT, ESPECIALLY SINCE IT IS DURING A VERY INTENSE PERIOD AT WORK. |
| PAT2 | 27/02/2020 | TEXT5 | MY DOG PASSED AWAY LAST WEEK. |
| PAT3 | 16/02/2020 | TEXT3 | I HAVE BEEN SEEING A THERAPIST FOR A WHILE NOW BUT SEE NO IMPROVEMENTS. MY WORK IS GETTING MORE AND MORE INTENSE. |
| PAT3 | 16/02/2020 | TEXT6 | THE PATIENT COMPLAINED ABOUT HEADACHES OVER THE PAST TWO WEEKS. |
| • • • • | • • • • | • • • • | • • • • |

## CLINICAL NOTES TABLE

# FIG. 6

600

STRUCTURED DATA

UNSTRUCTURED DATA
(TEXTUAL NOTES)

INFORMATIONAL
VALUE

INFORMATIONAL
ELEMENT

| PATIENT | WEEK | GENDER | AGE | MARITAL STATUS | NUMBER VISITS | DIAGNOSIS | NUMBER DAYS HOSPITALIZED | CRISIS | NOTES |
|---|---|---|---|---|---|---|---|---|---|
| PAT1 | WEEK1 | FEMALE | 52 | SINGLE | 1 | F31.0 | 5 | YES | TEXT1 TEXT2 |
| PAT2 | WEEK1 | MALE | 45 | MARRIED | 0 | NULL | 0 | NO | NULL |
| PAT3 | WEEK1 | MALE | 37 | INFORMAL RELATIONSHIP | 0 | NULL | 0 | NO | NULL |
| PAT1 | WEEK2 | FEMALE | 52 | SINGLE | 1 | F31.0 | 4 | YES | NULL |
| PAT2 | WEEK2 | MALE | 45 | MARRIED | 1 | NULL | 2 | NO | NULL |
| PAT3 | WEEK2 | MALE | 37 | INFORMAL RELATIONSHIP | 0 | NULL | 0 | NO | TEXT3 |
| PAT1 | WEEK3 | FEMALE | 52 | SINGLE | 0 | F31.0 | 0 | NO | NULL |
| PAT2 | WEEK3 | MALE | 45 | MARRIED | 0 | NULL | 7 | YES | TEXT4 TEXT5 |
| PAT3 | WEEK3 | MALE | 37 | INFORMAL RELATIONSHIP | 2 | F20.6 | 0 | NO | TEXT6 |
| • • • • | • • • • • | • • • • • | • • | • • • • | • • • • | • • • • | • • • • | • • | • • • |

DATA COMPILATION TABLE

FIG. 7

I HAVE BEEN HAVING ROUGH DAYS AT WORK AND FEEL BURNT OUT. THE ← 9    STARTING TEXTUAL NOTE (UNSTRUCTURED DATA)
WORK IS SO INTENSE THAT SOMETIMES I HAVE BEEN WANTING TO QUIT.

PREPROCESS TO REMOVE STOP WORDS
AND SPECIAL CHARACTERS. DO STEMMING.

ROUGH WORK FEEL BURNT WORK INTENSE SOMETIMES WANT QUIT ← 10    TOKENIZED CORPUS

APPLY A MODEL AND LIBRARY (FOR EXAMPLE, fastText) TO
CONVERT EACH TOKEN INTO A 60-DIMENSIONAL VECTOR

```
[-1.7373422e-03  8.0634799e-04 -3.7855241e-03  1.8606468e-03
 -2.2541641e-03 -2.8023494e-03  2.9186285e-03  3.7672662e-03
  9.9799491e-04  4.7291359e-03 -2.6433833e-03  2.6821948e-03
  1.0347718e-03 -1.5732923e-03  4.5456825e-04 -3.7201915e-03
  6.8020093e-04 -2.9759607e-04 -1.8646212e-03  1.3196677e-03
  4.0119910e-03  1.2802852e-04  5.1753032e-03 -2.0119848e-03
 -1.4626762e-03 -5.7291548e-04 -3.3977733e-03  2.7437699e-03
  2.9877743e-03  5.4034279e-03 -3.8716779e-03  2.0738912e-03
 -1.4522390e-03  5.8854823e-03 -3.4189872e-03  1.4847136e-03
  6.7496492e-04  3.5223151e-05 -1.3316668e-03  3.9780433e-03
 -5.0136005e-03 -2.8316365e-04 -3.0904007e-04 -3.4631069e-03
  1.0956305e-03 -3.5378295e-03 -9.1589446e-04  3.7757147e-03
 -5.3903594e-04  9.9411409e-04 -1.8644450e-03  3.1963827e-03
 -1.0405953e-04  2.6590910e-03  5.1750802e-04  3.8394055e-03
  4.0787449e-03  1.8068232e-03  2.0742591e-03 -3.9895992e-03]
```

← 11    THE 60-DIMENSIONAL VECTOR FOR THE TOKEN "ROUGH"

## CONVERT EACH TEXTUAL NOTE INTO A STRING OF 60-DIMENSIONAL VECTOR VALUES

# FIG. 8

900

| QUALITY ASSESSMENT OF STRUCTURED DATA | QUALITY ASSESSMENT OF UNSTRUCTURED DATA | MODEL SELECTED |
|---|---|---|
| LOW | LOW | MODEL#1 (XGBoost Decision Tree) |
| LOW | HIGH | MODEL#2 (Keras Neural Network) |
| HIGH | LOW | MODEL#1 (XGBoost Decision Tree) |
| HIGH | HIGH | MODEL#2 (Keras Neural Network) |

## MODEL SELECTION

# FIG. 11

700

| PATIENT | PCM (PERCENTAGE OF COLUMN WEEKS THAT ARE MISSING FOR THE PATIENT) | QUALITY LOW IF PCM >80% MEDIUM IF 50%<PCM<80% HIGH IF PCM<50% |
|---------|------|------|
| PAT1 | 16% | HIGH |
| PAT2 | 34% | HIGH |
| PAT3 | 22% | HIGH |
| PAT4 | 52% | MEDIUM |
| PAT5 | 8% | HIGH |
| PAT6 | 36% | HIGH |
| PAT7 | 42% | HIGH |
| PAT8 | 82% | MEDIUM |
| PAT9 | 12% | HIGH |
| PAT10 | 27% | HIGH |
| • • • • | • • • • | • • • • |

MORE THAN A THRESHOLD PERCENTAGE (70%) OF THE PATIENTS HAVE A "HIGH" QUALITY ASSESSMENT FOR STRUCTURED DATA, THEREFORE STRUCTURED DATA WILL BE USED IN SUBSEQUENT MODEL TRAINING STEP

QUALITY ASSESSMENT FOR
STRUCTURED DATA

FIG. 9

800

| PATIENT | PNM (PERCENTAGE OF WEEKS WITHOUT A NOTE) | QUALITY LOW IF PNM >80% MEDIUM IF 50%<PNM<80% HIGH IF PNM<50% |
|---------|------|------|
| PAT1 | 42% | HIGH |
| PAT2 | 83% | LOW |
| PAT3 | 67% | MEDIUM |
| PAT4 | 75% | MEDIUM |
| PAT5 | 16% | HIGH |
| PAT6 | 67% | MEDIUM |
| PAT7 | 92% | LOW |
| PAT8 | 100% | LOW |
| PAT9 | 25% | HIGH |
| PAT10 | 42% | HIGH |
| • • • • | • • • • | • • • • |

LESS THAN A THRESHOLD PERCENTAGE (70%) OF THE PATIENTS HAVE A "HIGH" QUALITY ASSESSMENT FOR UNSTRUCTURED DATA, THEREFORE UNSTRUCTURED DATA WILL NOT BE USED IN SUBSEQUENT MODEL TRAINING STEP

QUALITY ASSESSMENT FOR
UNSTRUCTURED DATA

FIG. 10

50

51

STEP 1: DATA INGESTION

57 ← STRUCTURED DATA (RE MENTAL HEALTH PATIENTS)

58 ← UNSTRUCTURED DATA (RE MENTAL HEALTH PATIENTS)

52

STEP 2: ASSESSMENT OF DATA QUALITY

55

ASSIGN A PATIENT'S DATA TO A BIN BASED ON QUALITY OF DATA (% OF COLUMN WEEKS (PCM) MISSING FOR THE PATIENT)

ASSIGN A PATIENT'S DATA TO A BIN BASED ON QUALITY OF DATA (% OF WEEKS WITHOUT A NOTE (PNM) FOR THE PATIENT)

HIGH QUALITY | MEDIUM QUALITY | LOW QUALITY

HIGH QUALITY | MEDIUM QUALITY | LOW QUALITY

STRUCTURED DATA QUALITY ASSESSMENT (IS DATA FOR >70% OF THE PATIENTS HIGH QUALITY ?)

UNSTRUCTURED DATA QUALITY ASSESSMENT (IS DATA FOR >70% OF THE PATIENTS HIGH QUALITY ?)

FEATURE EXTRACTION (WHEN AND IF NECESSARY)

53

STEP 3: MODEL SELECTION

DETERMINE WHAT DATA TO USE
- STRUCT ONLY ?
- UNSTRUCT ONLY ?
- STRUCT & UNSTRUCT ?

SELECT MODEL (BASED ON QUALITY ASSESSMENT OF STRUCTURED DATA AND QUALITY ASSESSMENT OF UNSTRUCTURED DATA)

54

STEP 4: MODEL TRAINING (IF NECESSARY)

RECORDS OF STRUCTURED DATA

STRINGS OF 60-D VECTOR VALUES

IF THE SELECTED MODEL REQUIRES TRAINING THEN TRAIN THE SELECTED MODEL

56

STEP 5: FEATURE EXTRACTION AND MENTAL HEALTH CRISIS EVENT DETECTION

INCOMING REAL-TIME STRUCTURED DATA

INCOMING REAL-TIME UNSTRUCTURED DATA

USE THE SELECTED MODEL ON REAL-TIME INCOMING DATA TO PREDICT A MENTAL HEALTH CRISIS EVENT

OUTPUT ALERT OF THE MENTAL HEALTH CRISIS EVENT

SYSTEM FOR TRIGGERING
MENTAL HEALTHCARE SERVICES
FIG. 12

SYSTEM FOR TRIGGERING
MENTAL HEALTHCARE SERVICES

# FIG. 13

Europäisches
Patentamt

European
Patent Office

Office européen
des brevets

# EUROPEAN SEARCH REPORT

Application Number

EP 21 20 6949

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/090733 A1 (DIBARI MARIA [US] ET AL) 25 March 2021 (2021-03-25) * paragraphs [0015] - [0031], [0054], [0074] * | 1-20 | INV. G16H50/20 G16H50/30 G16H50/70 |
| A | US 2020/111570 A1 (TRAN PELU S [US] ET AL) 9 April 2020 (2020-04-09) * paragraphs [0024], [0025] * | 1 | |
| A | US 2018/046761 A1 (KNOPLIOCH JEROME FRANCOIS [FR] ET AL) 15 February 2018 (2018-02-15) * paragraphs [0072], [0076] * | 1 | |

TECHNICAL FIELDS
SEARCHED      (IPC)

G16H

The present search report has been drawn up for all claims

| Place of search | Date of completion of the search | Examiner |
|---|---|---|
| Munich | 11 April 2022 | Rivera Pons, Carlos |

EPO FORM 1503 03.82 (P04C01)

## ANNEX TO THE EUROPEAN SEARCH REPORT
## ON EUROPEAN PATENT APPLICATION NO.

EP 21 20 6949

This annex lists the patent family members relating to the patent documents cited in the above-mentioned European search report.
The members are as contained in the European Patent Office EDP file on
The European Patent Office is in no way liable for these particulars which are merely given for the purpose of information.

11-04-2022

| Patent document cited in search report | | Publication date | Patent family member(s) | | Publication date |
|---|---|---|---|---|---|
| US 2021090733 | A1 | 25-03-2021 | NONE | | |
| US 2020111570 | A1 | 09-04-2020 | AU | 2019356784 A1 | 29-04-2021 |
| | | | EP | 3864587 A1 | 18-08-2021 |
| | | | US | 2020111570 A1 | 09-04-2020 |
| | | | WO | 2020076736 A1 | 16-04-2020 |
| US 2018046761 | A1 | 15-02-2018 | US | 2017091385 A1 | 30-03-2017 |
| | | | US | 2018046761 A1 | 15-02-2018 |
| | | | US | 2019172576 A1 | 06-06-2019 |

EPO FORM P0459

For more details about this annex : see Official Journal of the European Patent Office, No. 12/82